# EUROPÄISCHE PATENTANMELDUNG

(11) **EP 4 659 718 A1**
(43) Veröffentlichungstag der Anmeldung: **10.12.2025**
(21) Anmeldenummer: 24020182.2
(22) Anmeldetag: 06.06.2024
(51) Int. Cl.: A61F 5/01

(54) **THERAPEUTISCHES, INSBESONDERE ORTHOPÄDISCHES, HILFSMITTEL**

(71) Anmelder: medi GmbH & Co. KG, 95448 Bayreuth (DE)
(72) Erfinder: Bauer, Patrick, 95445 Bayreuth (DE); Zinke, Patrik, 95497 Goldkronach (DE)

(57) **Zusammenfassung**

Therapeutisches, insbesondere orthopädisches, Hilfsmittel (1, 2, 3) umfassend zumindest einen textilen Grundkörper (4, 5, 6) mit zumindest zwei zum Schließen des Grundkörpers (4, 5) miteinander verbindbare Endstücke (7, 8; 9, 10) und/ oder mit zumindest einem an dem Grundkörper (6) angebrachten textilen Gurtabschnitt (11, 12, 13), der zumindest ein Endstück (14, 15, 16) zum Befestigen des Gurtabschnitts (11, 12, 13) auf sich selbst oder an dem Grundkörper (5, 6) aufweist, wobei zumindest eines der Endstücke (7, 8; 9, 10; 14, 15, 16) zur Herstellung einer zugfesten Verbindung als textiles Flächengebilde (17, 17', 17", 17‴, 17"", 18; 19, 20; 21) mit zumindest einer ersten Materiallage (22, 23, 24) und zumindest einem an dem textilen Flächengebilde (17, 17', 17", 17‴, 17"", 18; 19, 20; 21) befestigten Verbindungselement (25, 26) ausgebildet ist und wobei zur Ausbildung zumindest einer einstückig mit der ersten Materiallage (22, 23, 24) ausgebildeten Lasche (27, 28, 29) zum Greifen des Endstücks (7, 8; 9, 10; 14, 15, 16) die zumindest eine erste Materiallage (22, 23, 24) zumindest zwei voneinander beabstandete erste Ausnehmungen (30, 31; 32, 33; 34, 35) aufweist.

## Beschreibung

Die vorliegende Erfindung bezieht sich auf ein therapeutisches, insbesondere orthopädisches, Hilfsmittel nach dem Oberbegriff des Anspruch 1.

Derartige therapeutische, insbesondere orthopädische, Hilfsmittel dienen insbesondere zur Stabilisierung, Entlastung, Ruhigstellung, Führung, Korrektur und/ oder Schützen von Gewebe, Gliedmaßen und/ oder des Rumpfes eines Patienten. Bandagen beispielweise werden speziell für verschiedene Körperteile, wie z.B. für den Oberkörper, insbesondere für den Rücken, oder für die Knie-, Ellenbogen- oder Handgelenke gefertigt und werden bei Entzündungen, Verletzungen oder präventiv getragen, um die entsprechenden Gelenke zu schonen, zu unterstützen oder diese vor erneuter Verletzung oder zu hoher Beanspruchung zu schützen. Zudem dienen derartige therapeutische Hilfsmittel aber auch dazu, Schwellungen, insbesondere Ödeme und Hämatome, abzubauen. Orthesen werden insbesondere zur zusätzlichen Stabilisierung und Entlastung von Gliedmaßen oder des Rumpfes eines Patienten eingesetzt. Durch das Tragen einer Lumbalorthese zum Beispiel wird der Bauchraum abgestützt und gleichzeitig die Wirbelsäule entlastet. Zudem können derartige Rückenorthesen zudem wirbelsäulenaufrichtende Funktionen aufweisen. Beispielsweise bei der Behandlung von Osteoporose, bei der durch die Abnahme der Knochendichte es zu einer Deformierung der Wirbelsäule und somit zu Fehlstellungen der Zwischenwirbelgelenke, Fehlfunktionen der Sehnen, Bänder und Muskulatur kommt, was Behinderungen im Alltag zur Folge hat, spielen diese Orthesen bzw. Eigenschaften eine wichtige Rolle.

Hierzu bestehen diese therapeutischen, insbesondere orthopädischen, Hilfsmittel üblicherweise aus einem textilen und elastischen Grundkörper. Dieser ist meist als kompressives Gestrick ausgebildet, das mittels einer Flach- oder Rundstrickmaschine flach- oder rundgestrickt ist. Insbesondere zur Stabilisierung und Ruhigstellung ist es ferner bekannt, dass diese Hilfsmittel neben dem elastischen textilen Grundkörper ein oder mehrere Stabilisierungselemente aufweisen, welche bevorzugt mit dem Grundkörper verbunden sind. Alternativ oder zusätzlich weisen die therapeutischen Hilfsmittel ein Gurtsystem zur zusätzlichen Stabilisierung und/ oder Ruhigstellung der Gliedmaßen und/ oder des Rumpfes des Patienten auf, welches bevorzugt ebenfalls an dem Grundkörper oder direkt an dem Stabilisierungselement angebracht ist.

Ein derartiges therapeutisches Hilfsmittel, insbesondere eine Rückenbandage, ist beispielsweise aus der EP 1 904 000 B1 bekannt.

Dabei besteht die bekannte Bandage aus mehreren Teilen, die zugfest miteinander verbindbar sind. Neben einem Mittelstück, nämlich einem Grundkörper, sind zwei zum Schließen der Bandage miteinander verbindbare Endstücke vorgesehen. Zur Herstellung einer zugfesten Verbindung zwischen den mehreren Teilen der Bandage weist nun ein Endstück ein flaches Ende mit beidseitig angebrachten Klettverschlusselementen auf und das zu verbindende andere Endstück ein maulartiges Ende, wobei an dessen Innenseite Klettverschlussgegenelemente angebracht sind. Um die Bandage um einen Rumpf herum mit einem gewissen Zug zu befestigen, weisen die Endstücke schließlich an ihrer Außenseite eine aufgesetzte Tasche auf, in die der Patient mit seinen Händen eingreifen kann.

Ein weiteres derartiges therapeutisches Hilfsmittel, insbesondere ebenfalls ausgebildet als Rückenbandage, ist beispielsweise aus der EP 2 822 515 B1 bekannt.

Dieser bekannte lumbale Stützgurt weist zwei getrennte Rückenteile auf, die v-förmig angeordnet sind und über ein elastisches Verbindungselement miteinander verbunden sind. Zwei Seitenteile erstrecken sich jeweils von den beiden Rückenteilen und werden in einem Bauchbereich des Benutzers zusammengefügt. Hierzu weist der Stützgurt zwei Endabschnitte auf. Zusätzlich sind elastische Bänder vorgehsehen, die ebenfalls v-förmig angeordnet sind und sich von einem mittleren Bereich des Verbindungselements bis zu den Endabschnitten erstrecken und an diesen befestigt sind. Außerdem weisen die seitlichen Endabschnitte zur Aufnahme der Finger des Benutzers jeweils eine Lasche auf, deren Enden an dem jeweiligen Endabschnitt der Bandage befestigt sind und unter der der Benutzer seine Finger hindurchführen kann.

Ein therapeutisches Hilfsmittel, ausgebildet als Rückenorthese zum Aufrichten der Wirbelsäule, ist beispielsweise aus der EP 3 659 561 A1 bekannt.

Diese aus dem Stand der Technik bekannte Orthese umfasst zum Stabilisieren des Rückens eines Patienten ein Rückenelement mit einem Stabilisierungskörper, das den Rücken abstützt, eine mit dem Rückenelement verbundene Hüftgurtanordnung sowie eine Schultergurtanordnung mit wenigstens zwei Schultergurtzügen. Die Schultergurtzüge erstrecken sich dabei von dem Rückenelement über die Schultern des Patienten bis zur Hüftgurtanordnung. Dabei sind die Schultergurtzüge zweiteilig ausgebildet und über ein Verbindungselement miteinander verbunden. Über das Verbindungselement ist die Gurtlänge in einem mittleren Bereich des Schultergurtzuges einstellbar. Ferner ist die Schultergurtanordnung mit der Hüftgurtanordnung über mindestens ein Führungselement verbunden. Das Führungselement ist dabei eine Umlenkschnalle mittels der die Länge des unteren Schultergurtabschnitts an dieser zweiten Position ebenfalls einstellbar ist. Die Hüftgurtanordnung weist eine Bauchpelotte auf, die mit der Schultergurtanordnung koppelbar oder gekoppelt ist, wobei bevorzugt die Bauchpelotte ein erstes und ein zweites Bauchpelottenelement umfasst, die miteinander verbindbar und voneinander trennbar sind. Dabei ist das erste und zweite Bauchpelottenelement über einen Klettverschluss lösbar miteinander verbindbar. Zum Greifen des ersten oder zweiten Bauchpelottenelements weisen diese eine Lasche auf. Dadurch können die Bauchpelottenelemente unter Zug miteinander verbunden werden, so dass die Hüftgurtanordnung fest in einem Hüftbereich an dem Patienten angelegt werden kann.

Als nachteilig bei diesen Ausgestaltungen der therapeutischen, insbesondere orthopädischen, Hilfsmittel erweist sich insbesondere die Ausbildung und Anbringung der Laschen an den ersten und/ oder zweiten Endstücken zur Herstellung einer Verbindung zwischen diesen unter Zug, insbesondere um die therapeutischen Hilfsmittel bspw. um einen Rumpf herum mit einem gewissen Zug zu befestigen.

Mit der Ausbildung der einen oder mehreren Laschen als zusätzliches Bauteil, welches an einem oder an beiden Endstücken befestigt, insbesondere aufgesetzt wird, ist ein deutlich erhöhter Produktionsaufwand verbunden. Das zusätzliche Bauteil, insbesondere die zusätzliche Materiallage, muss zunächst entsprechend der benötigten Form zugeschnitten und anschließend in einem eigenen Produktionsschritt an einem oder jeweils beiden Endstücken des therapeutischen Hilfsmittels befestigt werden, dies bspw. durch Aufkleben oder Aufnähen der Lasche auf zumindest eines der Endstücke.

Als nachteilig erweist sich dabei auch, dass durch die Ausbildung der Lasche als separates und zusätzliches Bauteil erhöhte Produktionskosten entstehen. Auch wenn der Zuschnitt der Lasche ggf. automatisiert erfolgen kann, so ist die Befestigung der Lasche an zumindest einem der Endstücke meist ein manueller Fertigungsschritt in einem Produktionsprozess. Jedenfalls sind mit der Herstellung der Lasche als zusätzliches Bauteil und der Befestigung der Lasche an einem oder an beiden Endstücken des therapeutischen Hilfsmittels Zusatzkosten verbunden.

Nachteilig ist ebenfalls, dass durch die Ausbildung der Lasche als zusätzliches Bauteil, insbesondere als zusätzliche Materiallage auf zumindest einem der Endstücke, der Tragekomfort des therapeutischen, insbesondere orthopädischen, Hilfsmittels verschlechtert wird. Zum einen trägt diese zusätzliche Materiallage zur Ausbildung der Lasche zusätzlich auf, so dass also das zumindest eine oder beide Endstücke einen vergrößerten Querschnitt aufweisen. Dies gilt es möglichst zu vermeiden. Patienten tragen derartige Hilfsmittel nämlich oftmals unter der Kleidung mit dem Wunsch, dass diese möglichst nicht zu erkennen sind. Hierzu ist es notwendig, dass die Hilfsmittel möglichst flach ausgebildet sind. Zum anderen führt aber auch die Anbringung bzw. das Aufsetzen einer zusätzlichen Materiallage auf dem zumindest einen oder auf beide Endstücke dazu, dass Kanten und insbesondere Ecken an dem zumindest einen Endstück, insbesondere an dessen Oberfläche, im Bereich der Befestigungspunkte der Lasche an dem Endstück entstehen. Diese Kanten und Ecken der zusätzlichen Materiallage können als störend empfunden werden, insbesondere dann, wenn bspw. die Ecken sich nach einem gewissen Nutzungszeitraum leicht von der Oberfläche des Endstücks lösen und leicht aufrichten. Dies ist bspw. bei Klebe- oder Nähverbindungen derartiger Textilflächen regelmäßig der Fall.

Ebenfalls als Nachteil erweist sich durch die Ausbildung der Lasche als separates Bauteil, insbesondere als zusätzliche Materiallage, welche auf zumindest einem der Endstücke aufgesetzt und mittels einer Fügetechnik an diesem befestigt ist, die schlechtere bzw. reduzierte Haltbarkeit der Lasche an dem zumindest einen Endstück. Eine Verbindung zweier separater Bauteile mittels einer Fügetechnik birgt immer die Gefahr, dass sich diese bei Zug voneinander lösen. So besteht die Gefahr, dass sich der Träger des therapeutischen Hilfsmittels beim Anlegen des Hilfsmittels unter Zug verletzt.

Der vorliegenden Erfindung liegt daher die Aufgabe zugrunde ein therapeutisches, insbesondere orthopädisches, Hilfsmittel bereit zu stellen, welches die Nachteile aus dem Stand der Technik vermeidet, welche also insbesondere einfach und kostengünstig in der Herstellung ist und gleichzeitig den Tragekomfort und die Haltbarkeit im Vergleich zu den aus dem Stand der Technik bekannten Lösungen weiter verbessert.

Gemäß einem Ausführungsbeispiel umfasst das erfindungsgemäße therapeutische, insbesondere orthopädische, Hilfsmittel aus zumindest einen textilen Grundkörper zumindest zwei zum Schließen des Grundkörpers miteinander verbindbare Endstücke und/ oder mit zumindest einem an dem Grundkörper angebrachten textilen Gurtabschnitt, der zumindest ein Endstück zum Befestigen des Gurtabschnitts auf sich selbst oder an dem Grundkörper aufweist, wobei zumindest eines der Endstücke zur Herstellung einer Verbindung unter Zug als textiles Flächengebilde mit zumindest einer ersten Materiallage und zumindest einem an dem textilen Flächengebilde befestigten Verbindungselement ausgebildet ist und wobei zur Ausbildung zumindest einer einstückig mit der ersten Materiallage ausgebildeten Lasche, insbesondere Schlaufe, zum Greifen des Endstücks, die zumindest eine erste Materiallage zumindest zwei voneinander beabstandete erste Ausnehmungen aufweist.

Bevorzugt besteht dabei der Grundkörper aus einem elastischen Gewebe oder Gewirke. Besonders bevorzugt ist der Grundkörper dabei als kompressives Gestrick ausgebildet, das auf einer Flachstrick- oder Rundstrickmaschine flach- oder rundgestrickt ist. In diesem Fall ist die Anbringung von Laschen bzw. Handschlaufen an den textilen Flächengebilde zum Greifen des oder der Endstücke besonders wichtig, um den Grundkörper unter Zug an dem Körperteil oder Rumpf eines Patienten anlegen zu können. Nur dann kann der Grundkörper des therapeutischen Hilfsmittels auch seine kompressive Wirkung enfalten.

Der zumindest eine Gurtabschnitt ist bevorzugt aus einem Velours gebildet. Dieser kann unlösbar an dem Grundkörper befestigt sein, bspw. über eine Verbindungsnaht. Alternativ kann dieser auch lösbar mit dem Grundkörper verbunden sein. Selbiges gilt auch für das zumindest eine textile Flächengebilde. Dieses kann ebenfalls unlösbar mit dem Grundkörper verbunden sein, oder von diesem lösbar sein. Dazu weist das textile Flächengebilde Befestigungsmittel auf, um dieses mit dem Grundkörper zu verbinden. Bevorzugt ist das zumindest eine Verbindungselement zumindest ein Klettelement eines Klettverschlusses. Auch das zuvor genannten Befestigungsmittel, um das zumindest eine textile Flächengebilde mit dem Grundkörper lösbar zu verbinden, kann als Klettelement eines Klettverschlusses ausgebildet sein.

Gemäß einem zweiten Ausführungsbeispiel des erfindungsgemäßen therapeutischen, insbesondere orthopädischen, Hilfsmittels sind die zumindest zwei voneinander beabstandeten ersten Ausnehmungen schlitzförmig ausgebildet. D.h. die Ausnehmungen sind lediglich durch zwei Einschnitte in der ersten Materiallage ausgebildet. Bevorzugt verlaufen beide Schlitze dabei parallel zueinander. Besonders bevorzugt sind diese zudem gewinkelt, insbesondere in einem Winkel zwischen 5 und 60 Grad, zu zumindest einer in einer Tragestellung gesehenen oberen oder unteren Außenkante des textilen Flächengebilde ausgebildet. Um nun das Risiko eines Einreißens der ersten Materiallage jeweils an den Enden der Schlitze zu verhindern, sind ferner erfindungsgemäß an diesen Stellen, also jeweils an den Enden der schlitzförmigen Ausnehmungen, bevorzugt kreisförmige Aussparungen vorgesehen.

Gemäß einem dritten Ausführungsbeispiel des erfindungsgemäßen therapeutischen, insbesondere orthopädischen, Hilfsmittels sind die zwei voneinander beabstandeten ersten Ausnehmungen durch flächige, insbesondere oval-förmige, Ausnehmungen ausgebildet. Bevorzugt verlaufen auch diese Ausnehmungen, insbesondere eine Mittelline der Ausnehmungen, in einem 90-Grad-Winkel oder gewinkelt, insbesondere in einem Winkel zwischen 5 Grad und 60 Grad, zu zumindest einer in einer Tragestellung gesehenen oberen oder unteren Außenkante des textilen Flächengebilde. Besonders bevorzugt beträgt der Winkel zwischen 10 und 45 Grad.

Gemäß einem weiteren Ausführungsbeispiel des erfindungsgemäßen therapeutischen, insbesondere orthopädischen, Hilfsmittels weist das textile Flächengebilde neben der ersten Materiallage eine zweite Materiallage auf. Die zweite Materialage bildet dabei bevorzugt eine Rückseite des textilen Flächengebilde, an welcher wiederum bevorzugt das eine oder die mehreren Verbindungselemente befestigt sind. Diese Materiallage ist besonders bevorzugt ebenfalls aus einem Gewebe, insbesondere einem Velours gebildet.

Gemäß einem weiteren Ausführungsbeispiel des erfindungsgemäßen therapeutischen, insbesondere orthopädischen, Hilfsmittels weist das textile Flächengebilde zumindest eine erste Zwischenlage, vorzugsweise eine Stabilisierungslage, auf, welche zwischen der erste und der zweiten Materiallage angeordnet ist. Dabei ist gemäß diesem Ausführungsbeispiel die erste und zweite Materiallage des textilen Flächengebilde bevorzugt aus einem Gewebe, insbesondere einem Velours, und die erste Zwischenlage, insbesondere die Stabilisierungslage, aus einem Kunststoff, insbesondere einem Polyurethan, gebildet.

Gemäß einem weiteren Ausführungsbeispiel des erfindungsgemäßen therapeutischen, insbesondere orthopädischen, Hilfsmittels weist das textile Flächengebilde zumindest zwei Zwischenlagen auf, wobei zwischen der ersten Materiallage, insbesondere im Bereich der Lasche, und einer ersten Zwischenlage, insbesondere der Stabilisierungslage, eine zweite Zwischenlage, insbesondere eine Dekorlage, angeordnet ist. Dabei ist gemäß diesem Ausführungsbeispiel die erste und zweite Materiallage des textilen Flächengebilde bevorzugt aus einem Gewebe, insbesondere einem Velours, die erste Zwischenlage, insbesondere die Stabilisierungslage, aus einem Kunststoff, insbesondere einem Polyurethan, und die zweite Zwischenlage, insbesondere die Dekorlage, ebenfalls aus einem Gewebe, insbesondere einem Velours oder aus einem Kunststoff, insbesondere aus einem Polyamid, gebildet.

Besonders bevorzugt weist dabei zumindest eine oder die mehreren Lagen und das zumindest eine Verbindungselement jeweils mehrere bevorzugt kreisförmige zweite Ausnehmungen auf, so dass die zumindest eine oder die mehreren Lagen und das Verbindungselement über bevorzugt stiftförmige Positionierungshilfen positionsgenau zueinander positionierbar sind. Die exakte Positionierung spielt insbesondere bei der Verbindung der mehreren Lagen, also der Fertigung des textilen Flächengebildes, insbesondere mittels einer Klebeverbindung, eine wichtige Rolle. Durch die Positionierungshilfen können die mehreren Lagen und Teile leicht und schnell exakt zueinander positioniert werden. Dies spart somit Zeit und Kosten bei der Produktion dieser textilen Flächengebilde.

Gemäß einem weiteren Ausführungsbeispiel des erfindungsgemäßen therapeutischen, insbesondere orthopädischen, Hilfsmittels ist die zumindest eine oder sind die mehreren Lagen und das zumindest eine Verbindungselement des textilen Flächengebilde durch eine oder mehrere Nähte, insbesondere Klebenähte, miteinander verbunden. Dabei handelt es sich besonders bevorzugt um eine stoffschlüssige Verbindung in Form einer Schweißverbindung. Hierbei wird eine aus Kunststoff bestehende oder mit Kunststoff kaschierte Lage lokal auf eine zu dieser benachbarten Lage aufgeschmolzen, so dass der aufgeschmolzene Kunststoff die benachbarte Lage benetzt, in diese gegebenenfalls geringfügig eindringt und sich beim Aushärten fest mit diesem verbindet. Alternativ können auch andere Fügetechniken zur Anwendung kommen, bspw. die Verwendung eines lokal oder flächig aufzutragenden Klebstoffes zur Verbindung der mehreren Lagen, oder das Vernähen der mehreren Lagen mittels eines Nähfadens.

Gemäß einem weiteren Ausführungsbeispiel des erfindungsgemäßen therapeutischen, insbesondere orthopädischen, Hilfsmittels weist die zumindest eine oder zumindest eine der mehreren Zwischenlagen zumindest eine bevorzugt mehrere dritte Ausnehmungen auf, so dass zwei zu dieser Lage benachbarte Lagen auch im Bereich der Zwischenlage unmittelbar miteinander verbindbar sind. D.h. die beiden zur Zwischenlage benachbarten Lagen können im Bereich der Ausnehmungen direkt in Kontakt treten. So ist es bspw. möglich diese beide benachbarten Lagen innerhalb der Zwischenlage direkt miteinander zu verbinden. Dazu kann ein Klebstoff verwendet werden. Alternativ ist es auch möglich, dass zumindest eine der beiden benachbarten Lagen aus einem Kunststoff gefertigt ist, oder zumindest damit kaschiert ist, welcher bei Energieeintrag schmilzt und die beiden zur Zwischenlage benachbarten Lagen im Bereich der dritten Ausnehmungen miteinander verbindet. Dadurch kann eine Verbindung dieser benachbarten Lagen insbesondere auch innerhalb der Zwischenlage erfolgen. Üblicherweise würde eine Verbindung dieser Lagen nur außerhalb der Zwischenlage erfolgen. Diese erfindungsgemäße Ausbildung des textilen Flächengebilde führt nun dazu, dass eine deutlich verbesserter Verbindung der mehreren Lagen miteinander erreicht wird, was die Haltbarkeit und Lebensdauer des textilen Flächengebilde und somit des medizinischen Hilfsmittel deutlich verbessert.

Gemäß einem weiteren Ausführungsbeispiel des erfindungsgemäßen therapeutischen, insbesondere orthopädischen, Hilfsmittels weist die zumindest eine oder zumindest eine der mehreren Lagen des textilen Flächengebilde zumindest eine Knicklinie auf, welche durch zumindest eine lokal begrenzte Materialschwächung in der oder den Lagen ausgebildet ist, so dass bevorzugt ein Randbereich des textilen Flächengebilde abknickbar ist. Bevorzugt weisen die Knicklinien dabei mehrere Abschnitte auf. D.h. die Materialschwächung ist zumindest bereichsweise unterbrochen. Die Knicklinien können geradlinig oder auch bogen- bzw. kurvenförmig auf dem textilen Flächengebilde, also auf einer oder den mehreren Lagen, verlaufen. Letzteres ist insbesondere im Bereich der Ecken des textilen Flächengebilde vorteilhaft, so dass diese Ecken in einem Tragezustand des Hilfsmittels abknickbar sind. Grundsätzlich hängt die Länge der jeweiligen Knicklinien von der Form bzw. Geometrie des jeweiligen textilen Flächengebilde ab und ist entsprechend zu wählen. Insbesondere wenn das textile Flächengebilde als Verschluss für einen Lumbalgürtel, insbesondere als Bauchpelotte, ausgebildet ist, spielen derartige Knicklinien eine besonders wichtige Rolle, da dadurch der Tragekomfort des therapeutischen Hilfsmittels, wesentlich verbessert wird. Es wird nämlich durch diese erfindungsgemäße Ausbildung ermöglicht, dass sich ein Randbereich des textilen Flächengebilde leicht abknicken lässt, insbesondre beim Sitzen, wenn sich die Bauchkontur zwangsläufig ändert. Es kommt also nicht mehr dazu, dass der Randbereich aufgrund seiner gewissen Steifigkeit am Bauch aufsteht.

Das therapeutische, insbesondere orthopädische, Hilfsmittel nach den zuvor beschriebenen Ausführungsbeispielen ist bevorzugt als Bandage, insbesondere als Rücken- oder Armbandage, oder als Orthese, insbesondere als Rücken- oder Rumpforthese, mit zumindest einem Stabilisierungselement ausgebildet.

Die vorliegende Sprunggelenkorthese zeichnet sich durch eine Reihe erheblicher Vorteile aus.

Durch die erfindungsgemäße Ausbildung des therapeutischen, insbesondere orthopädischen, Hilfsmittels wird insbesondere der Produktionsaufwand zur Herstellung eines derartigen therapeutischen Hilfsmittels mit einer Lasche zum Greifen eines Endstückes des Hilfsmittels, insbesondere unter Zug, verringert. Durch die einstückige Ausbildung der Lasche mit der zumindest einen Materiallage des textilen Flächengebilde, dies insbesondere durch die Ausbildung von zumindest zwei voneinander beabstandeten ersten Ausnehmungen in zumindest einer ersten Materiallage, ist es nicht mehr notwendig ein separates Bauteil anzufertigen und dieses anschließend an einem oder jeweils an beiden Endstücken des therapeutischen Hilfsmittels zu befestigen. Dadurch reduzieren sich auch die Herstellkosten für das therapeutische, insbesondere orthopädische, Hilfsmittel. Die Produktionskosten für eine separate Lasche sowie das Anbringen dieser an einem Endstück des therapeutischen Hilfsmittels entfallen nämlich.

Einen weiteren Vorteil der Erfindung stellt, neben dem verringerten Produktions- und Kostenaufwand, wie zuvor bereits geschrieben, der verbesserte Tragekomfort dar. Durch die einstückige Ausbildung der Lasche mit der zumindest einen Materiallage des textilen Flächengebilde wird ein besonders flaches Endstück an dem therapeutischen, insbesondere orthopädischen, Hilfsmittel bereitgestellt. Im Vergleich zum Stand der Technik kann nämlich dadurch die Bauhöhe eines derartigen textilen Flächengebilde um eine Materiallage, nämlich jene welche für die Ausbildung der Lasche benötigt wird, reduziert werden, welche üblicherweise auf das textile Flächengebilde zusätzlich aufgesetzt ist. Das therapeutische Hilfsmittel wird somit äußerst flach ausgebildet, so dass es gegenüber den vorbekannten Lösungen in einem Tragezustand weniger aufträgt. Zusätzlich wird aber auch der Tragekomfort dadurch verbessert, weil nun keine störenden Kanten und hervorstehende Ecken mehr, welche insbesondere durch die aufgesetzte Materiallage bzw. Lasche an dem zumindest einen Endstück gebildet werden, vorhanden sind.

Vorteilhaft ist dabei zudem, dass die Haltbarkeit des therapeutischen, insbesondere orthopädischen, Hilfsmittels deutlich verbessert wird. Durch die einstückige bzw. einteilige Ausbildung der Lasche mit der zumindest einen Materiallage des textilen Flächengebilde wird die Gefahr des Abreißens bzw. Lösen der Lasche von dem textilen Flächengebilde deutlich verringert, insbesondere dann, wenn durch den Anwender Zug auf eines der Endstücke des therapeutischen Hilfsmittels aufgebracht wird. Zusätzliche Befestigungsmittel, um die Lasche an der ersten Materiallage zu befestigen, bspw. in Form von Nähten, sind nämlich nicht vorhanden und werden auch nicht benötigt. Derartige zusätzlichen Befestigungsmittel stellen immer eine Schwachstelle bei einer Verbindung zweier Bauteile dar. Zudem wird damit auch das Risiko vermieden, zumindest deutlich reduziert, dass sich der Träger des therapeutischen Hilfsmittels beim Anlegen des Hilfsmittels unter Zug verletzt.

Nachfolgend wird die Erfindung anhand mehrerer Ausführungsbeispiele und in Verbindung mit den beigefügten Zeichnungen erläutert.

Dabei zeigen:
Figur 1 ein erstes Ausführungsbeispiel des erfindungsgemäßen therapeutischen, insbesondere orthopädischen, Hilfsmittels, insbesondere in Form einer Rückenbandage,
Figur 2 ein zweites Ausführungsbeispiel des erfindungsgemäßen therapeutischen, insbesondere orthopädischen, Hilfsmittels, insbesondere ausgebildet als Rückenorthese,
Figur 3 ein drittes Ausführungsbeispiel des erfindungsgemäßen therapeutischen, insbesondere orthopädischen, Hilfsmittels, insbesondere ausgebildet als Armbandage,
Figuren 4A bis 4E mehrere, insbesondere fünf, Ausführungsbeispiele des zumindest einen an dem Grundkörper des therapeutischen, insbesondere orthopädischen, Hilfsmittels gemäß Figur 1 angebrachten textilen Flächengebilde, insbesondere zur Herstellung einer Verbindung zwischen zwei Endstücken unter Zug,
Figur 5 das fünfte Ausführungsbeispiel des textilen Flächengebilde des erfindungsgemäßen therapeutischen, insbesondere orthopädischen, Hilfsmittels aus der Figur 4E in einer Schnittdarstellung;

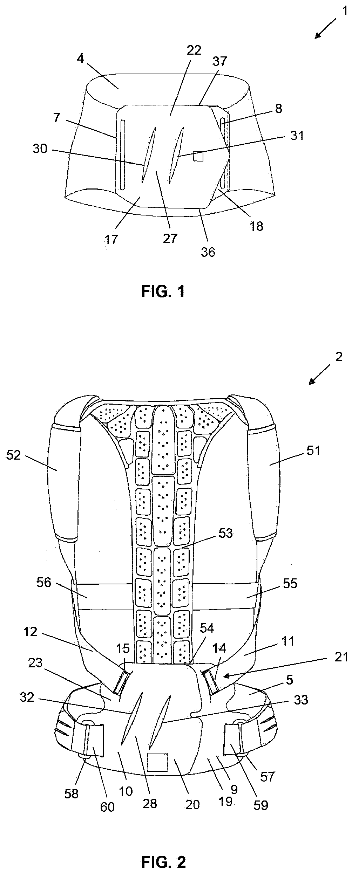

Wie in der Figur 1 zu sehen ist, weist ein erstes Ausführungsbeispiel des erfindungsgemäßen therapeutischen, insbesondere orthopädischen, Hilfsmittels, insbesondere die Rückenbandage 1, einen textilen Grundkörper 4 auf, der zumindest zwei zum Schließen des Grundkörpers 4 miteinander verbindbare Endstücke 7, 8 umfasst. Der gurtartige Grundkörper 4 ist dabei bevorzugt aus einem kompressiven und somit hoch elastischen Gestrick gebildet. Die beiden an diesem Grundkörper 4 bevorzugt unlösbar angebrachten Endstücke 7, 8 sind bevorzugt aus einem textilen Flächengebilde 17, 18 gebildet, das jeweils aus zumindest einer ersten Materiallage 22 besteht. Die Endstücke 7, 8 sind dabei bevorzugt unlösbar, bspw. mittels einer Nähnaht, mit dem Grundkörper 4 verbunden. Diese erste Materiallage 22 weist wiederum bevorzugt ein textiles Gewebe, insbesondere Velours auf. Besonders bevorzugt ist dieses als Doppelvelours ausgebildet, so dass die Vorder- und Rückseite der ersten Materiallage 22 aus einem Velours besteht. An der Rückseite des ersten textilen Flächengebilde 17 sowie an der Vorderseite des zweiten textilen Flächengebilde 18 ist ferner jeweils bevorzugt zumindest ein Verbindungselement befestigt, um die beiden Endstück 7, 8 der Rückenbandage 8 lösbar miteinander zu verbinden. Dabei bilden die beiden Verbindungselemente jeweils ein Klettelement eines Klettverschlusses aus.

Zur Herstellung einer Verbindung zwischen den beiden Endstücken 7, 8 und zum Schließen der Rückenbandage 1 unter Zug, so dass die Bandage 1 eng an dem Körper eines Trägers anliegt und die kompressive Wirkung entfalten kann, weist nun zumindest eines der beiden Endstücke 7, 8, also eines der beiden textilen Flächengebilde 17, 18, insbesondere das Flächengebilde 17 mit der ersten Materiallage 22, eine Lasche 27 zum Greifen des Endstücks 7 auf. Die Lasche 27 ist dabei durch zumindest zwei voneinander beabstandete erste Ausnehmungen 30, 31 in der ersten Materiallage 22 ausgebildet. Hierdurch wird die Lasche 27 einstückig bzw. einteilig mit und in der ersten Materiallage 22 gebildet. Die zumindest zwei ersten Ausnehmungen 30, 31 weisen dabei bevorzugt eine flächige, insbesondere oval-förmige, Form auf. Alternativ können diese auch durch zwei Schlitze in der ersten Materiallage 22 ausgebildet werden. Um nun das Greifen der Lasche 27, insbesondere das Hindurchführen der Finger eines Trägers zu erleichtern, sind die beiden Ausnehmungen 30, 31 bevorzugt gewinkelt, insbesondere in einem Winkel zwischen 5 und 60 Grad, besonders bevorzugt in einem Winkel zwischen 10 und 45 Grad, zu zumindest einer in einer Tragestellung gesehenen oberen oder unteren Außenkante 36, 37 des textilen Flächengebilde 17 angeordnet.

In der Figur 2 ist nun ein zweites Ausführungsbeispiel des erfindungsgemäßen therapeutischen, insbesondere orthopädischen, Hilfsmittels, insbesondere ausgebildet als Rückenorthese 2 gezeigt. Die Rückenorthese 2 umfasst dabei ebenfalls einen textilen und hüftgurtartigen Grundkörper 5, der ebenfalls zumindest zwei zum Schließen des Grundkörpers 5 miteinander verbindbare Endstücke 9, 10 umfasst. Die beiden Endstücke 9, 10 sind dabei bevorzugt jeweils lösbar mittels eines Umlenkelements 57, 58, welches insbesondere als D-Ring ausgebildet und jeweils an einer Schlaufe 59, 60 befestigt ist, mit dem Grundkörper 5, welcher bevorzugt aus einem Gewebe besteht, verbunden. Dadurch ist die Länge des Grundkörpers 5 einstellbar. Die lösbar angebrachten Endstücke 9, 10 sind dabei bevorzugt ebenfalls aus einem textilen Flächengebilde 19, 20 gebildet, das jeweils aus zumindest einer ersten Materiallage 23 besteht.

Auch in diesem Ausführungsbeispiel des therapeutischen, insbesondere orthopädischen, Hilfsmittels ist die zumindest eine erste Materialage 23 bevorzugt aus einem textilen Gewebe, insbesondere Velours, gebildet und weist erfindungsgemäß eine Lasche 28 zum Greifen des Endstücks 9 auf. Die Lasche 28 ist ebenfalls durch zumindest zwei voneinander beabstandete erste Ausnehmungen 32, 33 einstückig mit und in der ersten Materiallage 23 gebildet. Die beiden Endstücke 9, 10 können somit unter Zug miteinander verbunden werden, so dass der Grundkörper 5 der Rückenorthese 1 eng an dem Körper eines Trägers anliegt.

Zum Schließen der Rückenorthese 2 weisen die Endstück 9, 10, insbesondere die textilen Flächengebilde 19, 20, jeweils ebenfalls bevorzugt zumindest ein Verbindungselement, insbesondere ein Klettelement eines Klettverschlusses, auf.

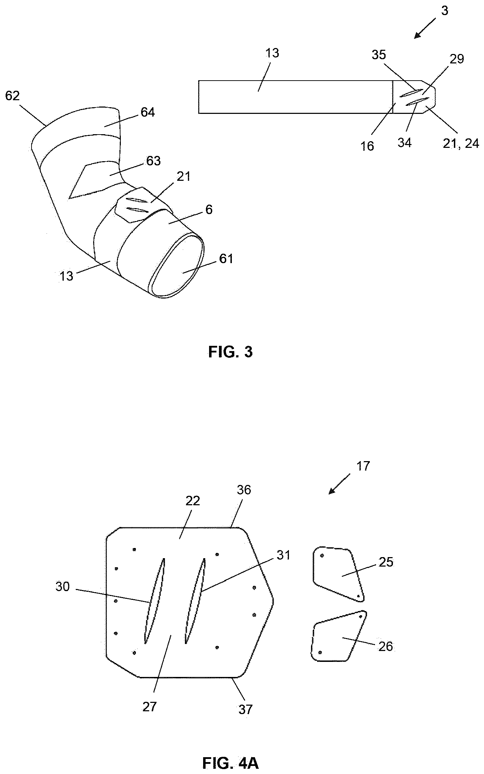

Neben dem Grundkörper 5 sowie der beiden Endstücke 9, 10, welche zusammen eine Bauchpelotte 54 bilden, weist die gezeigte Rückenorthese 2 nun ferner ein Rücken- und Stabilisierungselement 53 auf, welches mit dessen unterem Ende mit dem Grundkörper 5 verbunden ist. Von dem oberen Ende des Rückenelements 53 erstrecken sich zwei Schultergurte 51, 52, welche wiederum bevorzugt über zwei Umlenkgurte 55, 56 zu der Bauchpelotte 54, also zu den Endstücken 9, 10, geführt werden und dort vorzugsweise lösbar und in ihrer Länge einstellbar befestigbar sind. Hierzu weisen die unteren textilen Gurtabschnitte 11, 12 jeweils ein Endstück 14, 15 auf, das an der Bauchpelotte 54 befestigbar ist. Auch diese Endstücke 14, 15 der Gurtabschnitte 11, 12 weisen nun jeweils erfindungsgemäß bevorzugt eine Lasche zur Herstellung einer Verbindung unter Zug zwischen den beiden Endstücken 14, 15 und der Bachpelotte 54 auf. Die Ausbildung dieser Endstücke 14, 15, insbesondere des textilen Flächengebilde 21, entspricht der in der folgenden Figur 3 beschriebenen Ausbildung des Endstücks 16.

Figur 3 zeigt ein drittes Ausführungsbeispiel des erfindungsgemäßen therapeutischen, insbesondere orthopädischen, Hilfsmittels, insbesondere ausgebildet als Armbandage 3, welche sich in einem Tragezustand von einem unteren Oberarmbereich über den Ellenbogen bis in einen oberen Unterarmbereich erstreckt. Die Armbandage 3 weist dabei einen in Umfangsrichtung geschlossen bzw. hülsenförmigen textilen Grundkörper 6 mit einem offenem unteren und offenem oberen Ende 61, 62 auf. Der Grundkörper 6 ist dabei bevorzugt als kompressives Gestrick ausgebildet, dass neben einen Bund- oder Haftabschnitt 64 eine Komfortzone 63 umfasst. Die Komfortzone 63 hat dabei den Zweck die Ellenbeuge zu entlasten. Hierzu weist der Grundkörper 6 lokal begrenzt eine geringere Kompression auf, als im übrigen Gestrick. Zur Einstellung des Drucks im Unterarmbereich weist nun die Armbandage 3 zusätzlich einen bevorzugt abnehmbaren Gurtabschnitt 13 auf. Dieser ermöglicht die individuelle Dosierung des Drucks im oberen Unterarmbereich.

Zum Anbringen und Schließen des Gurtabschnitts 13 an dem Grundkörper 6 unter Zug weist nun der Gurtabschnitt 13, welche bevorzugt aus einem Gewebe, insbesondere Velours besteht, ein Endstück 16 auf, welches als textiles Flächengebilde 21 ausgebildet ist. Dieses besteht aus zumindest einer ersten Materiallage 24, welche wiederum eine Lasche 29 zum Greifen des Endstücks 16 aufweist. Die Lasche 29 wird dabei ebenfalls durch zumindest zwei voneinander beabstandete erste Ausnehmungen 34, 35 einstückig mit und in der ersten Materiallage 24 gebildet. Um das Greifen der Lasche 29, insbesondere das Hindurchführen der Finger eines Trägers zu erleichtern, sind auch die beiden Ausnehmungen 34, 35 bevorzugt gewinkelt, insbesondere in einem Winkel zwischen 5 und 60 Grad, besonders bevorzugt in einem Winkel zwischen 10 und 45 Grad, zu zumindest einer in einer Tragestellung gesehenen oberen oder unteren Außenkante des textilen Flächengebilde 21 angeordnet. An der Rückseite des textilen Flächengebilde 21 ist ferner zumindest ein Verbindungselement, bevorzugt ein Klettverschlusselement, befestigt, um das Endstück 16 des Gurtabschnitts 13 auf sich selbst oder an dem Grundkörper 6 zu befestigen.

Die Figuren 4A bis 4E zeigen nun mehrere, insbesondere fünf, Ausführungsbeispiele des zumindest einen an dem Grundkörper 4 des therapeutischen, insbesondere orthopädischen, Hilfsmittels 1 gemäß Figur 1 angebrachten textilen Flächengebilde 17, insbesondere zur Herstellung einer Verbindung zwischen zwei textilen Flächengebilde unter Zug.

Dabei zeigt die Figur 4A ein erstes Ausführungsbeispiel, bei dem das Endstück, insbesondere das textile Flächengebilde 17, aus einer einzigen Materiallage 22 besteht. Wie bereits zu Figur 1 beschrieben, ist die Materiallage 22 bevorzugt aus einem textilen Gewebe, insbesondere Velours, gefertigt. Besonders bevorzugt ist dieses als Doppelvelours ausgebildet, so dass die Vorder- und Rückseite der ersten Materiallage 22 aus einem Velours besteht. An der Rückseite des textilen Flächengebilde 17 werden bevorzugt die beiden Verbindungselemente 25, 26 befestigt, welche bevorzugt als Klettelemente eines Klettverschlusses ausgebildet sind. Zur Ausbildung einer Lasche 27 an dem textilen Flächengebilde und zum Greifen des Endstücks weist nun das textile Flächengebilde 17, insbesondere die erste Materiallage 22, zwei voneinander beabstandete erste Ausnehmungen 30, 31 auf. Hierdurch wird die Lasche 27 einstückig bzw. einteilig mit und in der ersten Materiallage 22 gebildet. Um das Greifen der Lasche 27, insbesondere das Hindurchführen der Finger eines Trägers, zu erleichtern, sind die beiden Ausnehmungen 30, 31 bevorzugt gewinkelt, insbesondere in einem Winkel zwischen 5 und 60 Grad, besonders bevorzugt in einem Winkel zwischen 10 und 45 Grad, zu zumindest einer in einer Tragestellung gesehenen oberen oder unteren Außenkante 36, 37 des textilen Flächengebilde 17 angeordnet.

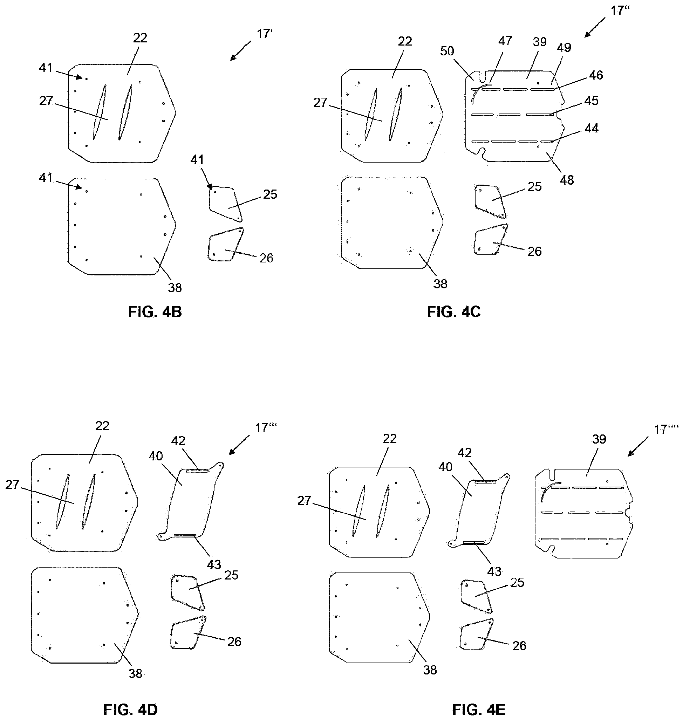

Figur 4B zeigt nun ein zweites Ausführungsbeispiel des zumindest einen an dem Grundkörper 4 des therapeutischen, insbesondere orthopädischen, Hilfsmittels 1 gemäß Figur 1 angebrachten textilen Flächengebilde 17`. Das Endstück, insbesondere das textile Flächengebilde 17' besteht dabei aus zwei Materiallagen 22, 38. Die zweite Materialage 38 bildet dabei die Rückseite des textilen Flächengebilde 17`. Die Verbindungselemente 25, 26 sind dabei an der zweiten Materiallage 38 befestigt. Die zweite Materiallage 38 dient im Wesentlichen dazu, die zumindest zwei ersten Ausnehmungen in der ersten Materiallage 22 zur Ausbildung der Lasche 27 rückseitig zu bedecken, so dass das Hindurchführen der Finger eines Trägers durch die Lasche 27 erleichtert wird. Die zweite Materiallage 38 verhindert nämlich, dass die Finger eines Trägers hinter das textile Flächengebilde 17` und somit hinter das Endstück gelangen.

Beide Materiallagen 22, 38 bestehen bevorzugt aus einem Gewebe, insbesondere aus einem Velours und sind dabei bevorzugt über mehrere Nähte, insbesondere Klebenähte, miteinander verbunden. Dabei handelt es sich besonders bevorzugt um eine stoffschlüssige Verbindung in Form einer Schweißverbindung. Bevorzugt ist hierzu zumindest eine der Materiallagen 22, 38 mit Kunststoff kaschiert, um diese Lage lokal auf die andere Lage aufzuschmelzen, so dass der aufgeschmolzene Kunststoff die andere Lage benetzt, in diese gegebenenfalls geringfügig eindringt und sich beim Aushärten fest mit dieser verbindet. Die Verbindungselemente 25, 26 sind dabei bevorzugt vollständig aus einem verschweißbaren Kunststoff gefertigt. Um die mehreren Lagen 22, 38 und Verbindungselemente 25, 26 exakt zueinander positionieren zu können, insbesondere im Rahmen des zuvor genannten Fügeprozesses, insbesondere Klebe- oder Schweißprozesses, weisen die mehreren gezeigten Komponenten bevorzugt mehrere kreisförmige zweite Ausnehmungen 41 auf, so dass die mehreren Lagen 22, 38 und die Verbindungselemente 25, 26 über bevorzugt stiftförmige Positionierungshilfen eines Werkzeuges positionsgenau zueinander positionierbar sind. Dies erleichtert den Fügeprozess wesentlich, was Zeit und somit Kosten in der Fertigung des therapeutischen, insbesondere orthopädischen, Hilfsmittels spart.

Figur 4C zeigt nun ein drittes Ausführungsbeispiel des zumindest einen an dem Grundkörper 4 des therapeutischen, insbesondere orthopädischen, Hilfsmittels 1 gemäß Figur 1 angebrachten textilen Flächengebilde 17". Das Endstück, insbesondere das textile Flächengebilde 17" weist dabei neben einer ersten und zweiten Materiallage 22, 38, wobei erneut in der ersten Lage die Lasche 27 ausgebildet ist, eine Zwischenlage 39, insbesondere eine Stabilisierungslage 39 auf, welche zwischen der ersten und zweiten Materiallage 22, 38 angeordnet wird. Die Zwischenlage 39 dient dazu dem textilen Flächengebilde 17" bzw. Endstück eine gewisse Festigkeit bzw. Biegesteifigkeit zu verleihen. Dies spielt insbesondere dann eine Rolle, wenn die textilen Flächengebilde 17" großflächig ausgebildet sind, bspw. wie in der Figur 1 und 2 gezeigt. Beide Materiallagen 22, 38 bestehen bevorzugt auch in diesem Ausführungsbeispiel aus einem Gewebe, insbesondere aus einem Velours. Die Zwischenlage 39 ist dabei bevorzugt aus einem Kunststoff, insbesondere aus einem Polyurethan, gefertigt. Die mehreren Lagen 22, 38, 39 sowie die Verbindungselemente 25, 26 sind auch hier bevorzugt über mehrere Nähte, insbesondere Klebenähte, miteinander verbunden.

Um nun aber den Tragekomfort durch die zusätzliche Stabilisierungslage 39 nicht negativ zu beeinflussen, insbesondere wenn das textile Flächengebilde 17" als Verschluss für einen Rückenbandage 1 bzw. Lumbalgürtel, insbesondere als Bauchpelotte, ausgebildet ist, weist diese Zwischenlage 39 mehrere Knicklinien 44, 45, 46, 47 auf, welche durch lokal begrenzte Materialschwächungen in der Zwischenlage 39 ausgebildet sind. Dadurch ist der Randbereich 48, 49 des textilen Flächengebilde 17" abknickbar. Bevorzugt weisen die Knicklinien dabei mehrere Abschnitte auf. D.h. die Materialschwächung ist zumindest bereichsweise unterbrochen. Die Knicklinien 44, 45, 46 sind dabei geradlinig ausgebildet. Die Knickline 47 ist hingegen bevorzugt bogenförmig ausgebildet. Dies ist insbesondere im Bereich der Ecken, bspw. in der Ecke 50, des textilen Flächengebilde 17" vorteilhaft, so dass die Ecke 50 in einem Tragezustand des Hilfsmittels abknickbar ist.

Figur 4D zeigt nun ein viertes Ausführungsbeispiel des zumindest einen an dem Grundkörper 4 des therapeutischen, insbesondere orthopädischen, Hilfsmittels 1 gemäß Figur 1 angebrachten textilen Flächengebilde 17‴. Das Endstück, insbesondere das textile Flächengebilde 17" weist dabei neben einer ersten und zweiten Materiallage 22, 38, wobei auch hier in der ersten Lage 22 die Lasche 27 ausgebildet ist, erneut eine Zwischenlage 40 auf. Bei der Zwischenlage 40 handelt es sich jedoch in diesem Ausführungsbeispiel um eine Dekorlage, welche zwischen der ersten und zweiten Materiallage 22, 38 angeordnet wird. Die Dekorlage 40 dient dazu die flächigen, insbesondere oval-förmigen Ausnehmungen zur Bildung der Lasche 27 sowie den Bereich unterhalb der Lasche 27 zu hinterlegen. Alle drei Materiallagen 22, 38, 40 bestehen bevorzugt in diesem Ausführungsbeispiel aus einem Gewebe, insbesondere aus einem Velours. Die mehreren Lagen 22, 38, 40 sowie die Verbindungselemente 25, 26 sind auch hier bevorzugt über mehrere Nähte, insbesondere Klebenähte, miteinander verbunden. Hierzu weist nun die Zwischenlage 40 zwei dritte Ausnehmungen 42, 43 auf, so dass die zwei zu dieser Lage benachbarten Lagen, nämlich die erste und zweite Lage 22, 38 auch im Bereich der Zwischenlage 40 miteinander verbindbar sind. D.h. die beiden zur Zwischenlage 40 benachbarten Lagen 22, 38 können im Bereich der Ausnehmungen 42, 43 direkt in Kontakt treten. So ist es möglich diese beide benachbarten Lagen 22, 38 innerhalb der Zwischenlage 40 direkt miteinander zu verbinden, bevorzugt mittels einer Klebeverbindung, insbesondere einer Schweißverbindung. Dazu ist die zweite Materiallage 38, welche aus einem Velours besteht, bevorzugt mit einem Kunststoff kaschiert, welcher bei Energieeintrag schmilzt und die Zwischenlage 40 im Bereich der dritten Ausnehmungen durchdringt und die erste Lage 22 direkt mit der zweiten Materiallage 38 stoffschlüssig verbindet. Dadurch kann eine Verbindung dieser Materiallagen 22, 38 auch innerhalb der Zwischenlage 40 erfolgen. Die dritten Ausnehmungen 42, 43 sind bevorzugt derart in der Zwischenlage 40 angeordnet, dass diese bei einer ordnungsgemäßen Positionierung der mehreren Lagen 22, 38, 40 zueinander im Bereich der Lasche 27, insbesondere an deren Anfang und Ende, zum Liegen kommen. Dadurch kann die erste Lage 22, insbesondere der Anfang und das Ende der ausgebildeten Lasche 27, mit der zweiten Lage 38 verbunden werden. Dies führt dazu, dass eine verbesserte Verbindung der mehreren Lagen 22, 38, 40 miteinander erreicht wird, was die Haltbarkeit und Lebensdauer des textilen Flächengebilde 17‴, aber insbesondere auch der Lasche 27, und somit des medizinischen Hilfsmittels 1 deutlich verbessert.

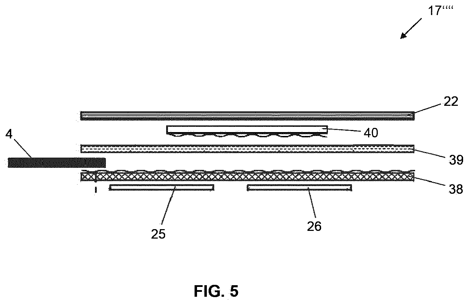

Figur 4E zeigt schließlich ein fünftes Ausführungsbeispiel des zumindest einen an dem Grundkörper 4 des therapeutischen, insbesondere orthopädischen, Hilfsmittels 1 gemäß Figur 1 angebrachten textilen Flächengebilde 17"". Das Endstück, insbesondere das textile Flächengebilde 17ʺʺ weist dabei neben einer ersten und zweiten Materiallage 22, 38 die zwei Zwischenlagen 39 und 40 auf. Neben der Stabilisierungslage 39 aus der Figur 4C ist ebenfalls eine Dekorlage 40, wie sie in der Figur 4D gezeigt ist, vorhanden. Die erste Materiallage 22 weist auch in diesem Ausführungsbeispiel die Lasche 27 auf. Die mehreren Lagen 22, 38, 39, 40 sowie die Verbindungselemente 25, 26 sind auch hier bevorzugt, wie bereits zu den vorherigen Ausführungsbeispielen beschrieben, über mehrere Nähte, insbesondere Klebenähte, miteinander verbunden. In der folgenden Figur 5 wird nun dieses fünfte Ausführungsbeispiel des textilen Flächengebilde 17ʺʺ des erfindungsgemäßen therapeutischen, insbesondere orthopädischen, Hilfsmittels in einer Schnittdarstellung gezeigt. Dabei wird auch die bevorzugte Materialauswahl der mehreren Lagen 22, 38, 39, 40 sowie deren Verbindung miteinander im Detail beschrieben.

Wie in der Figur 5 zu sehen ist, weist das textile Flächengebilde 17ʺʺ eine oberste Materiallage 22 auf. Diese Lage 22 bildet in einem Tragezustand die Außenseite des textilen Flächengebilde 17ʺʺ bzw. des Endstücks des therapeutischen, insbesondere orthopädischen, Hilfsmittels und weist dabei die Lasche 27 auf. Diese Lage besteht gemäß einem bevorzugen Ausführungsbeispiel aus einem Doppelvelours, welches also an beiden Seiten eine veloursartige Oberfläche umfasst. Unterhalb der ersten Materiallage 22 ist die Zwischenlage 40, insbesondere die Dekorlage 40 abgebildet, welche die in der ersten Lage 22 eingebrachten ersten Ausnehmungen und die Fläche unterhalb der Lasche bedeckt. Diese Dekorlage 40 ist bevorzugt ebenfalls aus einem Velours gebildet, weist nun jedoch an deren Unterseite eine Kunststoffkaschierung auf. Unterhalb der Dekorlage 40 ist wiederum die Stabilisierungslage 39 angeordnet. Diese besteht bevorzugt aus einem verschweißbaren Kunststoff, nämlich aus einem Polyurethan. Die Materiallage 38 stellt die zweite Materiallage dar und bildet dabei die Rückseite des textilen Flächengebilde 17"". Diese Lage 38 besteht bevorzugt ebenfalls aus einem Gewebe, insbesondere Velours, welches ebenfalls mit Kunststoff kaschiert ist. Die Kaschierung ist hier an der Innenseite angebracht, also an jener Seite, die zur Stabilisierungslage 38 zeigt. Schließlich sind noch die Verbindungselemente 25 und 26 gezeigt, welche bevorzugt ebenfalls aus einem verschweißbaren Kunststoff bestehen.

Erfindungsgemäß ist es nun durch diese Ausbildung der mehreren Materiallagen 22, 38, 39, 40 und Verbindungselemente 25, 26 möglich, das textile Flächengebilde 17ʺʺ in einem einzigen Fügeprozess bzw. Schritt, insbesondere Klebe- oder Schweißprozess, herzustellen. Hierzu werden die mehreren Materiallagen 22, 38, 39, 40 und Verbindungselemente 25, 26 bevorzugt über die in Figur 4B beschriebene Ausnehmungen 41 und über die bevorzugt stiftförmigen Positionierungshilfen eines Werkzeuges positionsgenau zueinander positioniert. Anschließend erfolgt die stoffschlüssige Verbindung durch Energieeintrag. Dadurch werden folgende Klebe- bzw. Schweißverbindungen zwischen den mehreren Materiallagen 22, 38, 39, 40 und Verbindungselemente 25, 26 geschaffen:
- Die aus einem verschweißbaren Kunststoff gefertigten Verbindungselemente 25, 26 werden auf die Veloursseite der zweiten Materiallage 38 aufgeschmolzen.
- Die zweite Materiallage 38, insbesondere deren Kunststoffkaschierung an der Innenseite, verbindet sich stoffschlüssig mit der Stabilisierungslage 39.
- Die Dekorlage 40, insbesondere deren Kunststoffkaschierung, verbindet sich wiederum ebenfalls stoffschlüssig mit der Stabilisierungslage 39.
- Die aus einem verschweißbaren Kunststoff gefertigte Stabilisierungslage 39 wird auf die erste Materiallage 22, insbesondere auf deren veloursartige Oberfläche aufgeschmolzen. Dies erfolgt außerhalb der Dekorlage 40, aber auch im Bereich der Dekorlage 40, nämlich durch die dritten Ausnehmungen 42, 43, welche in der Dekorlage 40 ausgebildet sind.

Um nun das textile Flächengebilde 17ʺʺ mit dem Grundkörper 4 des therapeutischen, insbesondere orthopädischen, Hilfsmittels 1 unlösbar zu verbinden wird nun bevorzugt in dem zuvor genannten Fertigungsschritt der Grundkörper 4 zwischen der zweiten Materiallage 38 und der Stabilisierungslage 39 eingeschoben und dort bevorzugt ebenfalls stoffschlüssig mit dem textilen Flächengebilde 17ʺʺ verbunden. Alternativ oder zusätzlich kann der Grundkörper 4 auch an das textile Flächengebilde 17ʺʺ angenäht werden.

Die Erfindung ist nicht auf die beschriebenen Ausführungsbeispiele beschränkt, sondern umfasst alle Ausführungen, die das prinzipielle, sinngemäße Funktionsprinzip der Erfindung anwenden oder beinhalten. Des Weiteren sind alle Merkmale aller beschriebenen und dargestellten Ausführungsbeispiele miteinander kombinierbar.

## Patentansprüche

1. Therapeutisches, insbesondere orthopädisches, Hilfsmittel (1, 2, 3) umfassend zumindest einen textilen Grundkörper (4, 5, 6) mit zumindest zwei zum Schließen des Grundkörpers (4, 5) miteinander verbindbare Endstücke (7, 8; 9, 10) und/ oder mit zumindest einem an dem Grundkörper (6) angebrachten textilen Gurtabschnitt (11, 12, 13), der zumindest ein Endstück (14, 15, 16) zum Befestigen des Gurtabschnitts (11, 12, 13) auf sich selbst oder an dem Grundkörper (5, 6) aufweist, wobei zumindest eines der Endstücke (7, 8; 9, 10; 14, 15, 16) zur Herstellung einer Verbindung unter Zug als textiles Flächengebilde (17, 17`, 17", 17‴, 17ʺʺ, 18; 19, 20; 21) mit zumindest einer ersten Materiallage (22, 23, 24) und zumindest einem an dem textilen Flächengebilde (17, 17`, 17", 17‴, 17"", 18; 19, 20; 21) befestigten Verbindungselement (25, 26) ausgebildet ist, **dadurch gekennzeichnet, dass** zur Ausbildung zumindest einer einstückig mit der ersten Materiallage (22, 23, 24) ausgebildeten Lasche (27, 28, 29) zum Greifen des Endstücks (7, 8; 9, 10; 14, 15, 16) die zumindest eine erste Materiallage (22, 23, 24) zumindest zwei voneinander beabstandete erste Ausnehmungen (30, 31; 32, 33; 34, 35) aufweist.

2. Therapeutisches, insbesondere orthopädisches, Hilfsmittel nach Anspruch 1, **dadurch gekennzeichnet, dass** die zumindest zwei voneinander beabstandeten ersten Ausnehmungen schlitzförmig ausgebildet sind.

3. Therapeutisches, insbesondere orthopädisches, Hilfsmittel (1, 2, 3) nach Anspruch 1, **dadurch gekennzeichnet, dass** die zwei voneinander beabstandeten ersten Ausnehmungen (30, 31; 32, 33; 34, 35) durch flächige, insbesondere oval-förmige, Ausnehmungen ausgebildet sind.

4. Therapeutisches, insbesondere orthopädisches, Hilfsmittel (1, 2, 3) nach Anspruch 2 oder 3, **dadurch gekennzeichnet, dass** die Ausnehmungen (30, 31; 32, 33; 34, 35) in einem 90-Grad-Winkel oder gewinkelt, insbesondere in einem Winkel zwischen 5 Grad und 60 Grad, zu zumindest einer in einer Tragestellung gesehenen oberen oder unteren Außenkante (36, 37) des textilen Flächengebilde (17, 17`, 17", 17‴, 17"", 18; 19, 20; 21) verlaufen.

5. Therapeutisches, insbesondere orthopädisches, Hilfsmittel (1) nach einem der vorherigen Ansprüche, **dadurch gekennzeichnet, dass** das textile Flächengebilde (17`) neben der ersten Materiallage (22) eine zweite Materiallage (38) aufweist.

6. Therapeutisches, insbesondere orthopädisches, Hilfsmittel (1) nach Anspruch 5, **dadurch gekennzeichnet, dass** das textile Flächengebilde (17", 17‴) zumindest eine erste Zwischenlage (39, 40), vorzugsweise eine Stabilisierungslage (39), aufweist, welche zwischen der erste und zweiten Materiallage (22, 38) angeordnet ist.

7. Therapeutisches, insbesondere orthopädisches, Hilfsmittel (1) nach Anspruch 6, **dadurch gekennzeichnet, dass** das textile Flächengebilde (17"") zumindest zwei Zwischenlagen (39, 40) aufweist, wobei zwischen der ersten Materiallage 22), insbesondere im Bereich der Lasche (27), und einer ersten Zwischenlage (39), insbesondere der Stabilisierungslage, eine zweite Zwischenlage (40), insbesondere eine Dekorlage, angeordnet ist.

8. Therapeutisches, insbesondere orthopädisches, Hilfsmittel (1) nach einem der vorherigen Ansprüche, **dadurch gekennzeichnet, dass** die zumindest eine oder die mehreren Lagen (22, 23, 24; 38, 39, 40) und das zumindest eine Verbindungselement (25, 26) jeweils mehrere bevorzugt kreisförmige zweite Ausnehmungen (41) aufweisen, so dass die zumindest eine oder die mehreren Lagen (22, 23, 24; 38, 39, 40) und das Verbindungselement (25, 26) über bevorzugt stiftförmige Positionierungshilfen positionsgenau zueinander positionierbar sind.

9. Therapeutisches, insbesondere orthopädisches, Hilfsmittel (1, 2, 3) nach einem der vorherigen Ansprüche, **dadurch gekennzeichnet, dass** die zumindest eine oder die mehreren Lagen (22, 23, 24; 38, 39, 40) und das zumindest eine Verbindungselement (25, 26) des textilen Flächengebilde (17, 17`, 17", 17‴, 17"", 18; 19, 20; 21) durch eine oder mehrere Nähte, insbesondere Klebenähte, miteinander verbunden sind.

10. Therapeutisches, insbesondere orthopädisches, Hilfsmittel (1) nach einem der Ansprüche 6 bis 9, **dadurch gekennzeichnet, dass** die zumindest eine oder zumindest eine der mehreren Zwischenlagen (40) zumindest eine bevorzugt mehrere dritte Ausnehmungen (42, 43) aufweist, so dass zwei zu dieser Lage (40) benachbarte Lagen (22, 38, 39) auch im Bereich der Zwischenlage (40) unmittelbar miteinander, insbesondere durch eine Klebenaht, verbindbar sind.

11. Therapeutisches, insbesondere orthopädisches, Hilfsmittel (1) nach einem der vorherigen Ansprüche, **dadurch gekennzeichnet, dass** die zumindest eine oder zumindest eine der mehreren Lagen (39) zumindest eine Knicklinie (44, 45, 46, 47) aufweist, welche durch zumindest eine Materialschwächung in der oder den Lagen (39) ausgebildet ist, so dass bevorzugt ein Randbereich (48, 49) des textilen Flächengebilde (17" 17"") abknickbar ist.

12. Therapeutisches, insbesondere orthopädisches, Hilfsmittel (1, 2, 3) nach einem der vorherigen Ansprüche, **dadurch gekennzeichnet, dass** die erste und/ oder zweite Materiallage (22, 23, 24; 38) des textilen Flächengebilde (17, 17`, 17", 17‴, 17ʺʺ, 18; 19, 20; 21) aus einem Gewebe, insbesondere einem Velours, die erste Zwischenlage (39), insbesondere die Stabilisierungslage, aus einem Kunststoff, insbesondere einem Polyurethan, und die zweite Zwischenlage (40), insbesondere die Dekorlage, ebenfalls aus einem Gewebe, insbesondere einem Velours, oder aus einem Kunststoff, insbesondere aus einem Polyamid, besteht.

13. Therapeutisches, insbesondere orthopädisches, Hilfsmittel (1, 2, 3) nach einem der vorherigen Ansprüche, **dadurch gekennzeichnet, dass** der Grundkörper (4, 5, 6) aus einem Gewebe, Gewirke oder bevorzugt kompressiven Gestrick besteht.

14. Therapeutisches, insbesondere orthopädisches, Hilfsmittel (1, 2, 3) nach einem der vorherigen Ansprüche, **dadurch gekennzeichnet, dass** das zumindest eine Verbindungselement (25, 26) zumindest ein Klettelement eines Klettverschlusses umfasst.

15. Therapeutisches, insbesondere orthopädisches, Hilfsmittel (1, 2, 3) nach einem der vorherigen Ansprüche, **dadurch gekennzeichnet, dass** das therapeutische Hilfsmittel eine Bandage, insbesondere eine Rücken- oder Armbandage (1, 3), oder eine Orthese, insbesondere eine Rücken- oder Rumpforthese (2), mit zumindest einem Stabilisierungselement (53) ist.
